(19) Europäisches Patentamt
European Patent Office
Office européen des brevets

(11) EP 4 538 257 A1

(12) EUROPEAN PATENT APPLICATION
published in accordance with Art. 153(4) EPC

(43) Date of publication:
**16.04.2025 Bulletin 2025/16**

(21) Application number: **23819910.3**

(22) Date of filing: **09.06.2023**

(51) International Patent Classification (IPC):
***C07C 233/65*** *(2006.01)* ***A61K 31/166*** *(2006.01)*
***A61K 31/437*** *(2006.01)* ***A61P 35/00*** *(2006.01)*
***C07D 471/04*** *(2006.01)* ***C07D 519/00*** *(2006.01)*

(52) Cooperative Patent Classification (CPC):
**A61K 31/166; A61K 31/437; A61P 35/00; C07C 233/65; C07D 471/04; C07D 519/00**

(86) International application number:
**PCT/JP2023/021536**

(87) International publication number:
**WO 2023/238930 (14.12.2023 Gazette 2023/50)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **10.06.2022 JP 2022094747**

(71) Applicant: **Taiho Pharmaceutical Co., Ltd.**
**Chiyoda-ku, Tokyo 101-8444 (JP)**

(72) Inventor: **WADA, Shiro**
**Kodama-gun, Saitama 367-0241 (JP)**

(74) Representative: **Wächtershäuser & Hartz Patentanwaltspartnerschaft mbB**
**Weinstraße 8**
**80333 München (DE)**

# (54) ANALOGUES OF AZABICYCLIC COMPOUNDS

(57) Provided is a novel compound serving as an analogue to be removed from API or a preparation. Further provided is a reference standard of an analogue for use in the quality control of a medicament. Analogue 1: 3-ethyl-4-{4-[4-(1-methyl-1H-pyrazol-4-yl)-1H-imidazol-1-yl]-3,3'-di(propan-2-yl)-1'H-[1,4'-bipyrazolo[3,4-b]pyridin]-1'-yl}benzamide. Analogue 2: 3-ethyl-4-fluorobenzamide. Analogue 3: N-[1-(4-carbamoyl-2-ethylphenyl)-3-(propan-2-yl)-1H-pyrazolo[3,4-b]pyridin-4-yl]-3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide. Analogue 4: 3-ethyl-4-{$1^4$-[4-(1-methyl-1H-pyrazol-4-yl)-1H-imidazol-1-yl]-$1^3$,$2^3$,$3^3$-tri(propan-2-yl)-$3^1$H-[$1^1$,$2^4$:$2^1$,$3^4$-terpyrazolo[3,4-b]pyridin]-$3^1$-yl}benzamide. Analogue 5: 4,4'-(1H,1'H-[4,4'-biimidazole]-1,1'-diylbis{[3-(propan-2-yl)-1H-pyrazolo[3,4-b]pyridine-4,1-diyl]})bis(3-ethylbenzamide). Analogue 6: 4-{4,6-bis[4-(1-methyl-1H-pyrazol-4-yl)-1H-imidazol-1-yl]-3-(propan-2-yl)-1H-pyrazolo[3,4-b]pyridin-1-yl}-3-ethylbenzonitrile. Analogue 7: 4-{4,6-bis[4-(1-methyl-1H-pyrazol-4-yl)-1H-imidazol-1-yl]-3-(propan-2-yl)-1H-pyrazolo[3,4-b]pyridin-1-yl}-3-ethylbenzamide. Analogue 8: 4-[4-ethoxy-3-(propan-2-yl)-1H-pyrazolo[3,4-b]pyridin-1-yl]-3-ethylbenzamide. Analogue 9: 3-ethyl-4-[4-methoxy-3-(propan-2-yl)-1H-pyrazolo[3,4-b]pyridin-1-yl]benzamide.

EP 4 538 257 A1

## Description

### Field of the Invention

The present invention relates to an analogue of 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide (hereinafter, also referred to as compound 1) or a salt thereof, and a pharmaceutical composition for oral administration, comprising the compound 1 or the salt thereof as an active ingredient.

### Background of the Invnention

The quality of active pharmaceutical ingredients (hereinafter, also referred to as API) must meet standards set by the International Council for Harmonisation of Technical Requirements for Pharmaceuticals for Human Use (hereinafter, also referred to as ICH). Subjects which the standards apply to are impurities contained in API or preparations. Examples thereof include analogues, residual solvents, and residual metals. It is required for API or preparations that such impurities should be removed to a level equal to or less than a threshold.

For confirming that impurities have been removed from API or preparations, it is necessary to provide and detect these impurities as reference standards or standard materials. Analogues, among other impurities, are organic compounds such as by-products or degradants formed in API production processes, or products or degradants formed during preservation of API or preparations containing API, and can generally be detected by high-performance liquid chromatography, thin-layer chromatography, or the like.

Patent Literature 1 and Non Patent Literature 1 disclose that 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide (compound 1) is useful as a prophylactic agent and/or a therapeutic agent for cancer or the like based on a HSP90 inhibitory effect.

Patent Literature 2 discloses that compound 1 exhibits a combinatorial effect with various antitumor agents.

Patent Literature 3 discloses that compound 1 exhibits a combinatorial effect, particularly, with an immune checkpoint inhibitor.

Patent Literature 4 discloses a crystal form useful as a medicament of compound 1.

Patent Literature 5 discloses that compound 1 or the like is useful in the treatment of IDO expression-related disease.

However, Patent Literatures 1 to 5 and Non Patent Literature 1 neither disclose nor suggest an analogue for use in quality control in order to produce a large amount of API having suitable quality as a medicament for compound 1.

### Citation List

#### Patent Literature

Patent Literature 1: WO 2011/004610
Patent Literature 2: WO 2015/046498
Patent Literature 3: WO 2019/004417
Patent Literature 4: WO 2016/181990
Patent Literature 5: WO 2019/054465


#### Non Patent Literature

Non Patent Literature 1: J. Med. Chem. 2019, 62, 531-551

### Summary of the Invention

#### Problems to be Solved by the Invention

An object of the present invention is to find a compound 1-derived novel analogue contained in a sample containing 3-ethyl-4-{4-[4-(1-methyl-1H-pyrazol-4-yl)-1H-imidazol-1-yl]-3-(propan-2-yl)-1H-pyrazolo[3,4-b]pyridin-1-yl} benzamide (compound 1) or a salt thereof, and provide the novel analogue as a reference standard of an analogue for use in the quality control of a medicament. A further object of the present invention is to provide a pharmaceutical composition for oral administration, comprising compound 1 or a salt thereof which meets standards set by ICH.

Means for Solving the Problem

The present inventors have conducted diligent studies and consequently found a compound 1-derived analogue which can be used as a reference standard for confirming suitable quality as a medicament for compound 1 or a salt thereof.

Specifically, the present invention provides the following [1] to [25].

[1] An analogue or a salt thereof that is any of the following analogues 1 to 9 or salts thereof, or a combination of the analogue(s) and the salt(s), for use as a reference standard for controlling the quality of 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a salt thereof:

analogue 1: 3-ethyl-4-{4-[4-(1-methyl-1H-pyrazol-4-yl)-1H-imidazol-1-yl]-3,3'-di(propan-2-yl)-1'H-[1,4'-bipyrazolo[3,4-b]pyridin]-1'-yl}benzamide
analogue 2: 3-ethyl-4-fluorobenzamide
analogue 3: N-[1-(4-carbamoyl-2-ethylphenyl)-3-(propan-2-yl)-1H-pyrazolo[3,4-b]pyridin-4-yl]-3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide
analogue 4: 3-ethyl-4-{$1^4$-[4-(1-methyl-1H-pyrazol-4-yl)-1H-imidazol-1-yl]-$1^3$,$2^3$,$3^3$-tri(propan-2-yl)-$3^1$H-[$1^1$,$2^4$:$2^1$,$3^4$-terpyrazolo[3,4-b]pyridin]-$3^1$-yl}benzamide
analogue 5: 4,4'-(1H,1'H-[4,4'-biimidazole]-1,1'-diylbis{[3-(propan-2-yl)-1H-pyrazolo[3,4-b]pyridine-4,1-diyl]})bis(3-ethylbenzamide)
analogue 6: 4-{4,6-bis[4-(1-methyl-1H-pyrazol-4-yl)-1H-imidazol-1-yl]-3-(propan-2-yl)-1H-pyrazolo[3,4-b]pyridin-1-yl}-3-ethylbenzonitrile
analogue 7: 4-{4,6-bis[4-(1-methyl-1H-pyrazol-4-yl)-1H-imidazol-1-yl]-3-(propan-2-yl)-1H-pyrazolo[3,4-b]pyridin-1-yl}-3-ethylbenzamide
analogue 8: 4-[4-ethoxy-3-(propan-2-yl)-1H-pyrazolo[3,4-b]pyridin-1-yl]-3-ethylbenzamide
analogue 9: 3-ethyl-4-[4-methoxy-3-(propan-2-yl)-1H-pyrazolo[3,4-b]pyridin-1-yl]benzamide.

[2] The analogue or a salt thereof, or a combination of the analogue(s) and the salt(s) according to [1], wherein the analogue for use as a reference standard is any of the analogues 1 to 7.
[3] The analogue or a salt thereof, or a combination of the analogue(s) and the salt(s) according to [1], wherein the analogue for use as a reference standard is any of the analogues 1 to 3.
[4] The analogue or a salt thereof, or a combination of the analogue(s) and the salt(s) according to [1], wherein the analogue for use as a reference standard is any of the analogues 1 and 2.
[5] The analogue or a salt thereof, or a combination of the analogue(s) and the salt(s) according to [1], wherein the analogue for use as a reference standard is the analogue 1.
[6] A pharmaceutical composition for oral administration, comprising 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a salt thereof as an active ingredient, wherein a total content of the following analogues 1 to 9 is 1.0% or less:

analogue 1: 3-ethyl-4-{4-[4-(1-methyl-1H-pyrazol-4-yl)-1H-imidazol-1-yl]-3,3'-di(propan-2-yl)-1'H-[1,4'-bipyrazolo[3,4-b]pyridin]-1'-yl}benzamide;
analogue 2: 3-ethyl-4-fluorobenzamide;
analogue 3: N-[1-(4-carbamoyl-2-ethylphenyl)-3-(propan-2-yl)-1H-pyrazolo[3,4-b]pyridin-4-yl]-3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide;
analogue 4: 3-ethyl-4-{$1^4$-[4-(1-methyl-1H-pyrazol-4-yl)-1H-imidazol-1-yl]-$1^3$,$2^3$,$3^3$-tri(propan-2-yl)-$3^1$H-[$1^1$,$2^4$:$2^1$,$3^4$-terpyrazolo[3,4-b]pyridin]-$3^1$-yl}benzamide;
analogue 5: 4,4'-(1H,1'H-[4,4'-biimidazole]-1,1'-diylbis{[3-(propan-2-yl)-1H-pyrazolo[3,4-b]pyridine-4,1-diyl]})bis(3-ethylbenzamide);
analogue 6: 4-{4,6-bis[4-(1-methyl-1H-pyrazol-4-yl)-1H-imidazol-1-yl]-3-(propan-2-yl)-1H-pyrazolo[3,4-b]pyridin-1-yl}-3-ethylbenzonitrile;
analogue 7: 4-{4,6-bis[4-(1-methyl-1H-pyrazol-4-yl)-1H-imidazol-1-yl]-3-(propan-2-yl)-1H-pyrazolo[3,4-b]pyridin-1-yl}-3-ethylbenzamide;
analogue 8: 4-[4-ethoxy-3-(propan-2-yl)-1H-pyrazolo[3,4-b]pyridin-1-yl]-3-ethylbenzamide; and
analogue 9: 3-ethyl-4-[4-methoxy-3-(propan-2-yl)-1H-pyrazolo[3,4-b]pyridin-1-yl]benzamide.

[7] The pharmaceutical composition for oral administration according to [6], wherein a content of the analogue 1 is

0.30% or less, and all of individual contents of the analogues 2 to 9 are 0.10% or less.

[8] A pharmaceutical composition for oral administration, comprising 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide as an active ingredient, wherein a content of 3-ethyl-4-{4-[4-(1-methyl-1H-pyrazol-4-yl)-1H-imidazol-1-yl]-3,3'-di(propan-2-yl)-1'H-[1,4'-bipyrazolo[3,4-b]pyridin]-1'-yl}benzamide as analogue 1 is 0.30% or less.

[9] A pharmaceutical preparation for oral administration, comprising a pharmaceutical composition for oral administration according to any of [6] to [8].

[10] 3-Ethyl-4-{4-[4-(1-methyl-1H-pyrazol-4-yl)-1H-imidazol-1-yl]-3,3'-di(propan-2-yl)-1'H-[1,4'-bipyrazolo[3,4-b]pyridin]-1'-yl}benzamide or a salt thereof.

[11] 3-Ethyl-4-fluorobenzamide or a salt thereof.

[12] N-[1-(4-Carbamoyl-2-ethylphenyl)-3-(propan-2-yl)-1H-pyrazolo[3,4-b]pyridin-4-yl]-3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a salt thereof.

[13] 3-Ethyl-4-{$1^4$-[4-(1-methyl-1H-pyrazol-4-yl)-1H-imidazol-1-yl]-$1^3$,$2^3$,$3^3$-tri(propan-2-yl)-$3^1$H-[$1^1$,$2^4$:$2^1$,$3^4$-terpyrazolo[3,4-b]pyridin]-$3^1$-yl}benzamide or a salt thereof.

[14] 4,4'-(1H,1'H-[4,4'-Biimidazole]-1,1'-diylbis{[3-(propan-2-yl)-1H-pyrazolo[3,4-b]pyridine-4,1-diyl]})bis(3-ethylbenzamide) or a salt thereof.

[15] 4-{4,6-Bis[4-(1-methyl-1H-pyrazol-4-yl)-1H-imidazol-1-yl]-3-(propan-2-yl)-1H-pyrazolo[3,4-b]pyridin-1-yl}-3-ethylbenzonitrile or a salt thereof.

[16] 4-{4,6-Bis[4-(1-methyl-1H-pyrazol-4-yl)-1H-imidazol-1-yl]-3-(propan-2-yl)-1H-pyrazolo[3,4-b]pyridin-1-yl}-3-ethylbenzamide or a salt thereof.

[17] 4-[4-Ethoxy-3-(propan-2-yl)-1H-pyrazolo[3,4-b]pyridin-1-yl]-3-ethylbenzamide or a salt thereof.

[18] 3-Ethyl-4-[4-methoxy-3-(propan-2-yl)-1H-pyrazolo[3,4-b]pyridin-1-yl]benzamide or a salt thereof.

[19] A method for producing 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide, comprising suspending, heating, and stirring water-containing wet crystals of 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide in methyl acetate.

[20] The method for producing 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide according to [19], further comprising adding the suspension to a methyl acetate/ethanol mixed solvent, and suspending, heating, and stirring the mixture, after suspending, heating, and stirring water-containing wet crystals of 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide in methyl acetate.

[21] Use of any of the following analogues 1 to 9 or salts thereof, or a combination of the analogue(s) and the salt(s) as a reference standard for controlling the quality of 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a salt thereof:

analogue 1: 3-ethyl-4-{4-[4-(1-methyl-1H-pyrazol-4-yl)-1H-imidazol-1-yl]-3,3'-di(propan-2-yl)-1'H-[1,4'-bipyrazolo[3,4-b]pyridin]-1'-yl}benzamide;
analogue 2: 3-ethyl-4-fluorobenzamide;
analogue 3: N-[1-(4-carbamoyl-2-ethylphenyl)-3-(propan-2-yl)-1H-pyrazolo[3,4-b]pyridin-4-yl]-3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide;
analogue 4: 3-ethyl-4-{$1^4$-[4-(1-methyl-1H-pyrazol-4-yl)-1H-imidazol-1-yl]-$1^3$,$2^3$,$3^3$-tri(propan-2-yl)-$3^1$H-[$1^1$,$2^4$:$2^1$,$3^4$-terpyrazolo[3,4-b]pyridin]-$3^1$-yl}benzamide;
analogue 5: 4,4'-(1H,1'H-[4,4'-biimidazole]-1,1'-diylbis{[3-(propan-2-yl)-1H-pyrazolo[3,4-b]pyridine-4,1-diyl]})bis(3-ethylbenzamide);
analogue 6: 4-{4,6-bis[4-(1-methyl-1H-pyrazol-4-yl)-1H-imidazol-1-yl]-3-(propan-2-yl)-1H-pyrazolo[3,4-b]pyridin-1-yl}-3-ethylbenzonitrile;
analogue 7: 4-{4,6-bis[4-(1-methyl-1H-pyrazol-4-yl)-1H-imidazol-1-yl]-3-(propan-2-yl)-1H-pyrazolo[3,4-b]pyridin-1-yl}-3-ethylbenzamide;
analogue 8: 4-[4-ethoxy-3-(propan-2-yl)-1H-pyrazolo[3,4-b]pyridin-1-yl]-3-ethylbenzamide; and
analogue 9: 3-ethyl-4-[4-methoxy-3-(propan-2-yl)-1H-pyrazolo[3,4-b]pyridin-1-yl]benzamide.

[22] The use according to [21], wherein the analogue is any of the analogues 1 to 7.

[23] The use according to [21], wherein the analogue is any of the analogues 1 to 3.

[24] The use according to [21], wherein the analogue is any of the analogues 1 and 2.

[25] The use according to [21], wherein the analogue is the analogue 1.

Advantageous Effects of Invention

According to the present invention, quality suitable for a pharmaceutical composition comprising compound 1 or a salt thereof as an active ingredient can be retained or controlled by using a compound 1-derived analogue as a reference standard for quality control. Specifically, the present invention can provide very highly pure compound 1 and a salt thereof having suitable quality as a medicament.

Brief Description of Drawings

Figure 1 is a HPLC chromatogram of compound 1 in Reference Example 1.
Figure 2 is a HPLC chromatogram of a reference standard of analogue 1.
Figure 3 is a HPLC chromatogram of a reference standard of analogue 2.

Description of Embodiments

In the present invention, 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide (compound 1), also called TAS-116 or pimitespib, is a compound having a structure of Formula 1 given below. For example, a method described in Patent Literature 1 or Non Patent Literature 1 is known as a method for producing compound 1 or a salt thereof.

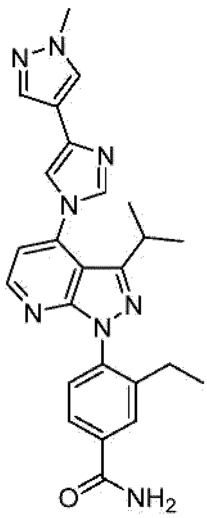

Compound 1

When the compound 1 has isomers such as optical isomers, stereoisomers, rotational isomers, or tautomers, any of the isomers and mixtures thereof are encompassed by the compound 1 unless otherwise specified.

The following Tables 1-1 to 1-3 show the compound names and structural formulas of compound 1-derived analogues 1 to 9 newly found in the present invention.

[Table 1-1]

| Analogue | Compound name | Structural formula |
|---|---|---|
| 1 | 3-Ethyl-4-{4-[4-(1-methyl-1H-pyrazol-4-yl)-1*H*-imidazol-1-yl]-3,3'-di(propan-2-yl)-1'*H*-[1,4'-bipyrazolo[3,4-b]pyridin]-1'-yl}benzamide | 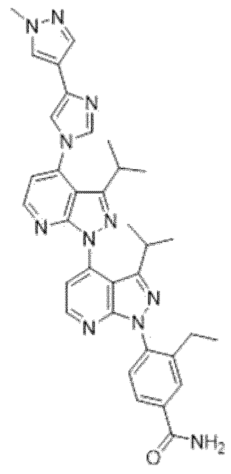 |
| 2 | 3-Ethyl-4-fluorobenzamide | 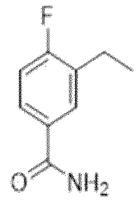 |
| 3 | *N*-[1-(4-Carbamoyl-2-ethylphenyl)-3-(propan-2-yl)-1H-pyrazolo[3,4-b]pyridin-4-yl]-3-ethyl-4-{4-[4-(1-methyl-1*H*-pyrazol-4-yl)-1H-imidazol-1-yl]-3-(propan-2-yl)-1*H*-pyrazolo[3,4-*b*]pyridin-1-yl}benzamide | 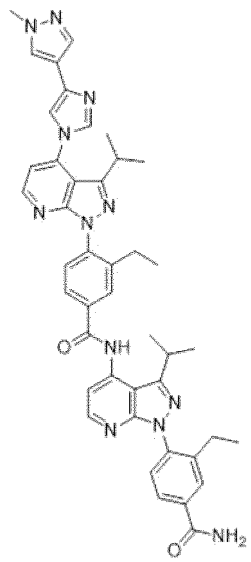 |

[Table 1-2]

| | | |
|---|---|---|
| 4 | 3-Ethyl-4-{$1^4$-[4-(1-methyl-1H-pyrazol-4-yl)-1*H*-imidazol-1-yl]-$1^3$,$2^3$,$3^3$-tri(propan-2-yl)-$3^1$*H*-[$1^1$,$2^{4-}2^1$,$3^4$-terpyrazolo[3,4-*b*]pyridin]-$3^1$-yl}benzamide | 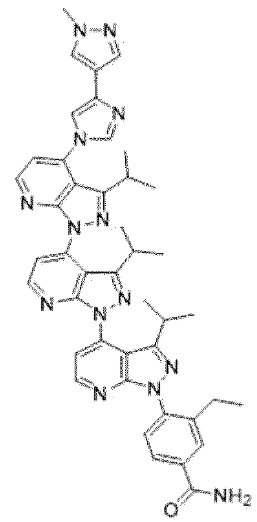 |
| 5 | 4,4'-(1*H,*1'*H*-[4,4'-Biimidazole]-1,1'-diylbis{[3-(propan-2-yl)-1H-pyrazolo [3,4-h]pyridine-4,1-diyl]})bis(3-ethylbenzamide) | 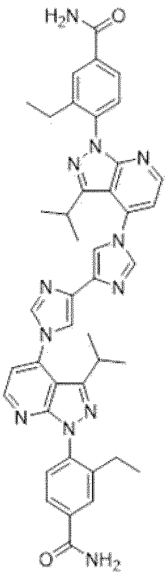 |
| 6 | 4-{4,6-Bis[4-(1-methyl-1*H*-pyrazol-4-yl)-1H-imidazol-1-yl]-3-(propan-2-yl)-1*H*-pyrazolo[3,4-h]pyridin-1-yl}-3-ethylbenzonitrile | 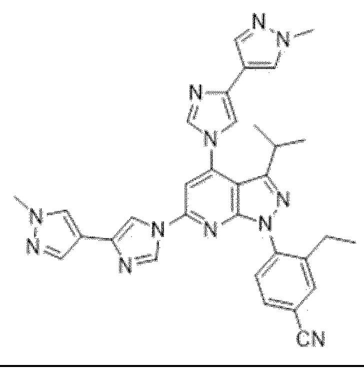 |

[Table 1-3]

| | | |
|---|---|---|
| 7 | 4-{4,6-Bis[4-(1-methyl-1*H*-pyrazol-4-yl)-1H-imidazol-1-yl]-3-(propan-2-yl)-1*H*-pyrazolo[3,4-*b*]pyridin-1-yl}-3-ethylbenzamide | 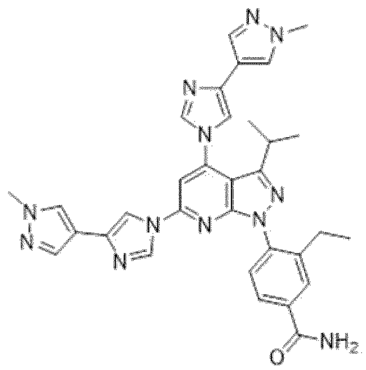 |
| 8 | 4-[4-Ethoxy-3-(propan-2-yl)-1*H*-pyrazolo[3,4-b]pyridin-1-yl]-3-ethylbenzamide | 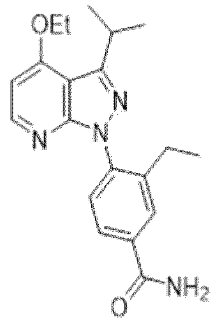 |
| 9 | 3-Ethyl-4-[4-methoxy-3-(propan-2-yl)-1*H*-pyrazolo[3,4-*b*]pyridin-1-yl] benzamide | 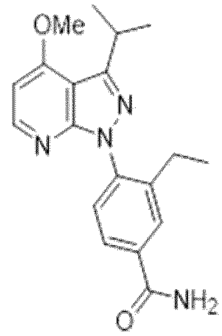 |

The analogue 1 is also referred to as DBA.
The analogue 2 is also referred to as FEBA.
The analogue 3 is also referred to as DBA-2.
The analogue 4 is also referred to as TBA.
The analogue 5 is also referred to as PZI-01-04.
The analogue 6 is also referred to as AZI-04-05.
The analogue 7 is also referred to as AZI-04-06.
The analogue 8 is also referred to as EOABA.
The analogue 9 is also referred to as MOABA.
All the analogues 1 to 9 are compounds which may be formed in the course of producing the compound 1. The analogues 1 to 9 and their salts can be regarded as impurities which may be contained in API and preparations of the compound 1.
As used herein, the terms "analogue 1", "analogue 2", "analogue 3", "analogue 4", "analogue 5", "analogue 6", "analogue 7", "analogue 8", and "analogue 9" can be meant to include "salts" and "solvates (e.g., hydrates)" of the analogues. The analogues 1 to 9 also include their tautomers.
As used herein, the salt means a pharmaceutically acceptable salt unless otherwise specified. Examples thereof can include base-addition salts and acid-addition salts.
Examples of the base-addition salt include: alkali metal salts such as sodium salt and potassium salt; alkaline earth metal salts such as calcium salt and magnesium salt; ammonium salts; and organic amine salts such as trimethylamine salt, triethylamine salt, dicyclohexylamine salt, ethanolamine salt, diethanolamine salt, triethanolamine

salt, procaine salt, and **N,N'-**dibenzylethylenediamine salt.

Examples of the acid-addition salt include: inorganic acid salts such as hydrochloride, sulfate, nitrate, phosphate, and perchlorate; organic acid salts such as acetate, formate, maleate, fumarate, tartrate, citrate, ascorbate, and trifluoroacetate; and sulfonates such as methanesulfonate, isethionate, benzenesulfonate, and p-toluenesulfonate.

The compound 1 or the salt thereof used in the present invention is preferably a free form of the compound 1 which has not formed a salt.

Batch production for use in the production of products or compounds is a method of adding a given amount of starting materials, and producing at once a certain amount of products or compounds while respective steps proceed sequentially.

Batch production using, for example, 1 kg or more as the amount of starting materials added in one production process called one batch, **i.e.,** using, for example, 1 kg or more of starting materials in producing desired compounds once, is referred to as large-scale production in the present embodiment. The desired compounds include API as well as intermediates for use in the production of API.

Whether the compound 1 or the salt thereof thus obtained sufficiently satisfies the quality of API is determined by analyzing the compound 1 or the salt thereof. This determination abides by, for example, standards described in ICH.

In this respect, a chemical purity serving as a standard is calculated by measurement such as high-performance liquid chromatography (hereinafter, also referred to as HPLC). An analogue can be identified by comparing a retention time, a mass spectrum, and a photodiode array (PDA) of high-performance liquid chromatography with those of the analogues 1 to 9 detected according to the present invention.

Further, these analogues 1 to 9 can also be quantitatively measured by any of a method using an external standard and a method using an internal standard.

When these analogues 1 to 9 may be contained as impurities in a medicament or a preparation, these analogues 1 to 9 are controlled in accordance with the guideline of the International Council for Harmonisation of Technical Requirements for Pharmaceuticals for Human Use (ICH-Q3). The analysis of the analogues 1 to 9 are very useful because whether a medicament or a preparation satisfies the standards of the guideline can be confirmed.

These analogues 1 to 9 can be detected from the compound 1 or the salt thereof by an analogue analysis method mentioned later. In the present invention, one or more of the analogues 1 to 9 can be used as reference standards in quality control. Preferably, the analogues 1 to 5 are used as reference standards in quality control. Further more preferably, the analogues 1 and 2 are used as reference standards in quality control. Most preferably, the analogue 1 is used as a reference standard in quality control.

Such a compound 1-derived analogue for use as a reference standard has a high purity. Thus, each of the analogues separated under conditions of high-performance liquid chromatography can be used as a reference standard. Accordingly, the present invention may be interpreted as a method for producing the analogue, comprising isolating the analogue from a sample containing compound 1 or a salt thereof. Examples of the analogue include the analogues 1 to 9 described above. The analogue is preferably the analogues 1 **to 5,** further more preferably the analogues 1 and **2,** most preferably the analogue 1.

It has not been known so far that the compound 1 may contain the analogues 1 to **9.** The analogues 1 and 3 to 9 are novel substances. Hence, the present invention includes analogues 1 to 9 for use in the quality control of an active pharmaceutical ingredient of compound 1 or a salt thereof or a preparation containing compound 1 or a salt thereof. The present invention further includes analogues 1 to 9 for use as reference standards in detecting impurities from a preparation containing compound 1 or a salt thereof.

<Analogue analysis method>

In the present embodiment, API (compound 1 or a salt thereof) and a preparation containing this API are to be measured for confirming the presence of the analogues 1 to 9 or their salts. The preparation containing API may contain, for example, a fluidizer, an excipient, a binder, a disintegrant, a lubricant, a coating agent, a colorant, a flavor, and a corrigent which are usually used.

The analogues 1 to 9 according to the present embodiment or their salts can be used as reference standards for controlling quality by confirming the stability of API or a preparation containing API, and the presence or absence of each analogue in the measurement of an infrared absorption (IR) spectrum, a nuclear magnetic resonance (NMR) spectrum, or chromatography such as HPLC or thin-layer chromatography (TLC) in a production process thereof.

For example, in the case of measuring an analogue in API or a preparation using an IR spectrum, the presence of the analogue in the API or the preparation can be confirmed provided that an absorption band derived from the compound 1 or the salt thereof, or an excipient or the like contained in the preparation does not overlap with a partial absorption band derived from the analogue or a salt thereof.

In the case of measuring an analogue in API or a preparation using an NMR spectrum, the presence of the analogue in the API or the preparation can be confirmed provided that a peak derived from the compound 1 or the salt

thereof, or an excipient or the like contained in the preparation does not overlap with a partial peak derived from the analogue or a salt thereof.

In the case of measuring an analogue in API or a preparation using HPLC, the presence of the analogue in the API or the preparation can be confirmed provided that a peak derived from the compound 1 or the salt thereof, or an excipient or the like contained in the preparation does not overlap in retention time with a partial peak derived from the analogue or a salt thereof.

In the case of measuring an analogue in API or a preparation using TLC, the presence of the analogue in the API or the preparation can be confirmed provided that a spot derived from the compound 1 or the salt thereof, or an excipient or the like contained in the preparation does not overlap in Rf value with a partial spot derived from the analogue or a salt thereof.

In the present embodiment, the presence of the analogues 1 to 9 or their salts in API or a preparation containing API can be confirmed by the measurement described above. Measurement by HPLC is preferred from the viewpoint that a very small amount of each analogue or a salt thereof can be measured.

The measurement described above requires providing a reference standard of each analogue or a salt thereof. The terms "reference standard" and "standard material" are given various definitions depending on each application of use in the Japanese Pharmacopoeia. In the present embodiment, the "reference standard" is a substance serving as an index for confirming the presence of an analogue contained in API or a preparation. Hence, the "reference standard" according to the present embodiment conceptually includes not only the reference standard but the standard material described in the Japanese Pharmacopoeia.

In the present embodiment, the reference standard of each analogue or a salt thereof may be isolated from API, a crude product, or an intermediate or the like in producing the compound 1 or the salt thereof, or may be separately synthesized.

Examples of the analogue which can be used as a reference standard for controlling quality include starting material or intermediate compounds, by-product compounds in the production of API, and compounds formed in producing preparations from API. The analogue is not particularly limited as long as the analogue is a compound which may be contained in actually produced API or preparations. The analogue may not be actually contained in API or a preparation. Thus, such an analogue from the viewpoint of API production includes not only a compound actually contained in API or a preparation but a compound removed in a purification process of API. The compound 1 or the salt thereof which may contain the analogue according to the present embodiment is not limited to those produced by the production method described above.

In the present embodiment, a commercially available product can usually be used in HPLC. HPLC involves at least a column for separation and a detector.

In the present embodiment, the content of each analogue contained in API or a preparation is calculated according to the following equation based on the peak area of the analogue in the measurement of HPLC.

$$\text{Content (\%) of the analogue} = \text{Peak area of the analogue} / \text{Total peak area} \times 100.$$

In the present embodiment, resolution Rs of a peak by the measurement of HPLC indicates the relationship between mutual retention times of peaks on a chromatogram and respective peak widths, and is calculated according to the following equation.

$$Rs = 1.18 \times (tR2 - tR1) / (W0.5h1 + W0.5h2)$$

tR1 and tR2: retention times of two substances used in resolution measurement wherein tR1 < tR2.
W0.5h1 and W0.5h2: peak widths at midpoints in the heights of respective peaks.
tR1 and tR2 or W0.5h1 and W0.5h2 are used at the same unit.

The Japanese Pharmacopoeia states that a resolution of 1.5 or more means a state in which a peak is completely separated. In the present embodiment, the resolution of 1.5 or more can also be used as an index.

In the present embodiment, measurement conditions for use in HPLC are not particularly limited as long as one analogue selected from the group consisting of the analogues 1 to 9 and the compound 1 have a resolution of 1.5 or more. The number of times of measurement of HPLC is not particularly limited as long as the measurement is performed once or more.

In the present embodiment, a gradient of a mobile phase, a silica gel column, an injection volume of a measurement sample, the presence or absence of a mobile phase cleaner, a measurement wavelength, a column temperature, a flow rate of the mobile phase, a mixer volume of a HPLC apparatus, and the like can be appropriately set as

measurement conditions for use in HPLC.

For HPLC, a normal-phase column which employs an organic phase as a mobile phase and separates a compound depending on its polarity, and a reverse-phase column which employs an aqueous phase as a mobile phase and separates a compound are known. In the present embodiment, reverse-phase chromatography using a reverse-phase column is preferred from the viewpoint of the physical properties of the compound 1.

The mobile phase will be mentioned later.

In general, in HPLC measurement, the point in time when a measurement sample is injected (introduced) to a mobile phase and detection in a detector is started is regarded as the start of measurement. Hereinafter, in the present embodiment, the point in time during HPLC measurement may be defined with reference to the start of measurement.

The HPLC column for separation which can be used in the present embodiment is selected from the group consisting of, for example, a silica gel column, a silica gel column surface-modified with an octadecylsilyl group (ODS column or C18 column), a silica gel column surface-modified with an octyl group (C8 column), a silica gel column surface-modified with a cyanopropyl group (CN column), a silica gel column surface-modified with a phenethyl group (Ph column), a silica gel column surface-modified with an aminopropyl group (NH column), a silica gel column surface-modified with a dihydroxypropyl group (Diol column), columns packed with various polymers (polymer columns), and a column packed with an ion exchange resin (ion exchange column), and is preferably an ODS column.

Various ODS columns can be used which differ in a particle size of a silica gel, a pore size, a binding method for the octadecylsilyl group, the degree of substitution of the octadecylsilyl group, and the like. In the present embodiment, a highly pure silica gel is used, and an ODS column in which residual silanol after octadecylation is treated with a low-molecular silylating agent (endcapped ODS column) is preferred.

Various ODS columns can be used which differ in a particle size of a silica gel, a pore size, a binding method for the octadecylsilyl group, the degree of substitution of the octadecylsilyl group, and the like.

The average particle size of the silica gel is more preferably on the order of, for example, from 3 to 5 $\mu$m. The average particle size of the silica gel can be measured by a laser diffraction method or the like.

The average pore size of the silica gel is more preferably, for example, from 10 to 12 nm. The average pore size of the silica gel can be measured by a gas adsorption method or the like.

The binding pattern of the octadecylsilyl group in the silica gel is preferably, for example, a monomeric pattern or a polymeric pattern.

The degree of substitution of the octadecylsilyl group can be measured by various methods. The amount of carbon in the silica gel is preferably, for example, 14% or more. The amount of carbon in the silica gel is preferably, for example, 20% or less. The amount of carbon in the silica gel can be measured by various methods.

A liquid mixture of an organic phase and an aqueous phase is preferably used as the HPLC mobile phase according to the present embodiment.

The organic phase for use in the HPLC mobile phase is a liquid medium composed mainly of an organic solvent. For example, a nonpolar solvent (e.g., hexane, cyclohexane, heptane, diethyl ether, tetrahydrofuran, chloroform, and methylene chloride), an aprotic polar solvent (e.g., acetone, dimethyl sulfoxide, and acetonitrile), acetic acid, methanol, ethanol, isopropanol, or acetonitrile can be used as the organic solvent. One of these organic solvents can be used singly, or two or more thereof can be used in combination (e.g., as a mixed solvent). The organic solvent contained in the organic phase according to the present embodiment is preferably acetonitrile.

The organic phase may also contain 50% by volume or less of water. Preferably, water constitutes 40% by volume or less, more preferably 30% by volume or less, particularly preferably 20% by volume or less, of the whole organic phase.

The aqueous phase for use in the HPLC mobile phase is a liquid medium composed mainly of water. The whole amount of the liquid contained in the aqueous phase can be water.

The aqueous phase may also contain 20% by volume or less of an organic solvent. The organic solvent for use in this case is not particularly limited as long as the organic solvent can be homogeneously mixed with water. Examples thereof include acetone, dimethyl sulfoxide, acetonitrile, formic acid, acetic acid, methanol, ethanol, and isopropanol and acetonitrile is preferred.

The organic solvent which may be contained in the aqueous phase constitutes 50% by volume or less, preferably 10 to 50% by volume, more preferably 20 to 40% by volume, of the whole aqueous phase.

The pH of the HPLC mobile phase is not particularly limited as long as the analogues 1 to 9 or their salts are detectable and the contents thereof can be calculated. The pH of the HPLC mobile phase can be adjusted by the addition of a buffer given below.

A buffer can be added to the HPLC mobile phase from the viewpoint of reducing the influence of pH on measurement and improving reproducibility. For example, formic acid or a salt thereof, acetic acid or a salt thereof, citric acid or a salt thereof, tartaric acid or a salt thereof, or phosphoric acid or a salt thereof can be added as the buffer.

Examples of the formic acid or the salt thereof include formic acid, sodium formate, and ammonium formate. Examples of the acetic acid or the salt thereof include acetic acid and sodium acetate. Examples of the citric acid or the salt

thereof include citric acid, monosodium citrate, disodium citrate, and trisodium citrate. Examples of the tartaric acid or the salt thereof include tartaric acid and sodium tartrate. Examples of the phosphoric acid or the salt thereof include phosphoric acid, sodium dihydrogen phosphate, disodium hydrogen phosphate, potassium dihydrogen phosphate, and dipotassium hydrogen phosphate. One of these buffers can be used singly, or two or more thereof can be used in combination.

The concentration of the buffer which can be used in the present invention can be appropriately adjusted to a concentration that prevents buffer precipitation during high-performance liquid chromatography measurement. The concentration is preferably from 1 to 50 mM, more preferably from 3 to 30 mM, further more preferably from 5 to 20 mM, particularly preferably from 8 to 15 mM.

The buffer can be added to both the aqueous phase and the organic phase. Preferably, the buffer is added to the aqueous phase.

The concentration of the buffer which can be used in the present embodiment can be appropriately adjusted to a concentration that prevents buffer precipitation during HPLC measurement.

In the present embodiment, the solvent and the buffer described above can be used in the aqueous phase in the mobile phase. The aqueous phase is preferably an aqueous solution containing formic acid or a salt thereof as the buffer, or an aqueous solution containing phosphoric acid or a salt thereof as the buffer, more preferably an aqueous solution of phosphate.

In general, in the measurement of HPLC, the analogue of interest can be properly separated by varying the liquid mixture composition of the organic phase and the aqueous phase in the mobile phase. In this respect, the analogue of interest can be measured by keeping a constant compositional ratio of the liquid mixture in the mobile phase (isocratic) or continuously changing the composition of the liquid mixture (gradient).

In reverse-phase chromatography, the varying liquid mixture composition of the organic phase and the aqueous phase in the mobile phase usually employs a two-dimensional graph of the ratio (%) of the organic phase in the whole mobile phase on the ordinate against a measurement time (min) on the abscissa. Specifically, for isocratic separation, the slope is represented by the linear function of 0. For a gradient in which the ratio of the organic phase is elevated over time in a constant increment, the slope is represented by a positive linear function. For a gradient in which the ratio of the organic phase is lowered over time in a constant decrement, the slope is represented by a negative linear function. These approaches can be combined so that measurement can be achieved for a short retention time by efficiently eluting even a low-polarity analogue while maintaining the resolution of each peak.

The increment of the organic phase in the mobile phase, which is the "slope" of the linear function, can be represented by, for example, an increment of the organic phase per unit time and can be calculated as follows.

Increment of the organic phase per unit time = {(Ratio of the organic phase in the mobile phase at the completion of the gradient) - (Ratio of the organic phase in the mobile phase at the start of the gradient)} / (Length of time between the start and completion of the gradient)

For example, when the ratio of the organic phase is 10% by volume of the mobile phase at the start of measurement (0 minutes) and is continuously increased so as to become 30% by volume of the mobile phase 10 minutes after the start of measurement, the increment of the organic phase per unit time is defined as 2% by volume/min.

In the present embodiment, the composition of the mobile phase and the presence or absence of change therein are not particularly limited as long as the analogues described above can be measured.

In the present embodiment, the measurement time of HPLC is not particularly limited as long as the analogue of interest can be measured. Examples thereof include 3 to 120 minutes. The measurement time is preferably from 10 to 60 minutes.

In the present embodiment, the ratio of the aqueous phase at the start of measurement of HPLC (0 minutes) is from 50 to 100% by volume of the whole mobile phase.

In the present embodiment, in the case of measuring API of the compound **1,** the ratio of the aqueous phase at the start of measurement of HPLC is from 50 to 100% by volume, preferably from 50 to 80% by volume, more preferably from 50 to 75% by volume, of the whole mobile phase.

In the present embodiment, the ratio of the aqueous phase at the completion of measurement of HPLC is from 0 to 50% by volume of the whole mobile phase.

In the present embodiment, in the case of measuring API of the compound **1,** the ratio of the aqueous phase at the completion of measurement of HPLC is from 0 to 50% by volume, preferably from 10 to 50% by volume, more preferably from 20 to 40% by volume, of the whole mobile phase.

The analogue of interest can be measured by appropriately adopting isocratic separation, a gradient, and a combination thereof from the start to completion of measurement of HPLC.

In the present embodiment, a solvent for use in the mobile phase described above can be adopted to a solution to be injected to the mobile phase at the start of measurement by HPLC. Water or a buffer solution for use in the aqueous phase in the mobile phase, acetonitrile, isopropanol, and a mixed solvent thereof are preferred.

In the present embodiment, the flow rate of the mobile phase in a column in measurement by HPLC is not particularly limited as long as the rate is measurable. The flow rate is preferably from 0.5 to 2.0 mL/min.

In the present embodiment, the detection wavelength in measurement by HPLC is not particularly limited as long as the wavelength is detectable. The detection wavelength is preferably from 210 to 400 nm, further more preferably 220 nm.

In the present embodiment, the amount of the solution to be injected to the mobile phase at the start of measurement by HPLC is not particularly limited as long as the amount is measurable. The amount is preferably 10 μL or more.

A mobile phase cleaner may be appropriately used in the HPLC according to the present embodiment. The cleaner employs active carbon in a cartridge and can remove impurities or garbage from the mobile phase. This reduces baseline noise of a chromatogram. Therefore, even a small amount of a compound 1-derived analogue can be properly detected and quantified.

In the present invention, the compound 1 or the salt thereof is mixed with a solvent to prepare a sample, which is then injected as a measurement sample to high-performance liquid chromatography so that a compound 1-derived analogue can be detected.

In the present invention, in the case of isolating the compound 1 at a high purity such that the total content of the analogues 1 to 9 is 1.0% or less, a purification step is preferably performed by the following crystallization.

First, water-containing wet crystals of the compound 1 obtained by a method comprising the steps described above are purified. The solvent which is adopted in the purification of the wet crystals of the compound 1 is preferably a C6 to C14 aromatic hydrocarbon, a C1 to C6 alcohol, a C1 to C6 ester, or a mixed solvent thereof, more preferably methyl acetate.

Before proceeding to the subsequent step, the obtained wet crystals of the compound 1 may be filtered or washed. The solvent which is adopted in filtration or washing is preferably water, a C6 to C14 aromatic hydrocarbon, a C1 to C6 alcohol, a C1 to C6 ester, or a mixed solvent thereof, more preferably an ethanol/water mixed solvent.

Subsequently, the obtained compound 1 is purified to obtain crystals of the compound 1. The solvent which is adopted in further purification of the compound 1 is preferably a C6 to C14 aromatic hydrocarbon, a C1 to C6 alcohol, a C1 to C6 ester, or a mixed solvent thereof, more preferably a methyl acetate/ethanol mixed solvent.

In the case of adopting a solvent containing methyl acetate/ethanol (mixed solvent) as the solvent which is adopted in further purification of the compound 1, the ratio of the volume of methyl acetate to the volume of ethanol as the solvent ratio of an initial methyl acetate/ethanol liquid mixture can be from 0.01 to 100, and is preferably from 0.1 to 50, more preferably from 0.5 to 25, further more preferably from 1.0 to 10.

The method for producing compound 1 according to the present embodiment comprises suspending, heating, and stirring water-containing wet crystals of the compound 1 in methyl acetate. The method further comprises adding the obtained compound 1 to a methyl acetate/ethanol mixed solvent, and suspending, heating, and stirring the mixture.

The method for producing compound 1 preferably comprises suspending, heating, and stirring water-containing wet crystals of the compound 1 in methyl acetate, and further comprises adding the obtained compound 1 to a methyl acetate/ethanol mixed solvent, and suspending, heating, and stirring the mixture, wherein a yield of the compound 1 per batch is 1 kg or more.

The method for producing compound 1 more preferably comprises suspending, heating, and stirring water-containing wet crystals of the compound 1 in methyl acetate, and further comprises adding the obtained compound 1 to a methyl acetate/ethanol mixed solvent, and suspending, heating, and stirring the mixture, wherein a yield of the compound 1 per batch is 1 kg or more.

The method for producing compound 1 further more preferably comprises suspending, heating, and stirring water-containing wet crystals of the compound 1 in methyl acetate, and further comprises adding the obtained compound 1 to a methyl acetate/ethanol mixed solvent, and suspending, heating, and stirring the mixture, wherein a yield of the compound 1 per batch is 5 kg or more.

The method for producing compound 1 even more preferably comprises suspending, heating, and stirring water-containing wet crystals of the compound 1 in methyl acetate, and further comprises adding the compound 1 to a methyl acetate/ethanol mixed solvent, and suspending, heating, and stirring the mixture, wherein a yield of the compound 1 per batch is 10 kg or more.

The method for producing compound 1 much more preferably comprises suspending, heating, and stirring the compound 1 in a solvent containing methyl acetate and ethanol in which the ratio of the volume of methyl acetate to the volume of ethanol is 0.01 to 100, wherein a yield of the compound 1 per batch is 10 kg or more.

The pharmaceutical composition for oral administration of the present invention comprises compound 1 or a salt thereof as an active ingredient. In the pharmaceutical composition for oral administration of the present invention, the total content of the analogues 1 to 9 is 1.0% or less, preferably 0.5% or less, from the viewpoint of meeting standards set by ICH. In the pharmaceutical composition for oral administration, the content of the analogue 1 is 0.30% or less. In the pharmaceutical composition for oral administration, all of the individual contents of the analogues 2 to 9 are 0.10% or less.

In the pharmaceutical composition for oral administration of the present invention, the total content of the analogues 1 to 9 is from 0% to 1.0%. In the pharmaceutical composition for oral administration, the content of the analogue

1 is from 0% to 0.30%. In the pharmaceutical composition for oral administration, all of the individual contents of the analogues 2 to 9 are from 0% to 0.10%.

In the pharmaceutical composition for oral administration of the present invention according to a preferred embodiment, the total content of the analogues 1 to 9 is more than 0% and 1.0% or less, preferably more than 0% and 0.5% or less. In the pharmaceutical composition for oral administration, the content of the analogue 1 is more than 0% and 0.30% or less. In the pharmaceutical composition for oral administration, all of the individual contents of the analogues 2 to 9 are more than 0% and 0.10% or less.

The pharmaceutical composition for oral administration of the present invention is blended, if necessary, with a pharmaceutically acceptable carrier and is capable of adopting various dosage forms depending on a prophylactic or therapeutic purpose. Such various dosage forms can each be produced by a common formulation method known to those skilled in the art.

Various common organic or inorganic carrier substances as pharmaceutical materials are used as the pharmaceutically acceptable carrier and blended as an excipient, a binder, a disintegrant, a lubricant, and a colorant for solid preparations or as a solvent, a solubilizer, a suspending agent, a tonicity agent, a buffer, a soothing agent, and the like for liquid preparations. A pharmaceutical additive such as an antiseptic, an antioxidant, a colorant, a sweetener, or a stabilizer may be used, if necessary.

The pharmaceutical composition for oral administration of the present invention is preferably a tablet containing compound 1 or a salt thereof, and an excipient selected from the group consisting of lactose, corn starch, and crystalline cellulose or a film-coated tablet obtained by film-coating the tablet, further more preferably a tablet containing compound 1 or a salt thereof, lactose, corn starch, and crystalline cellulose or a film-coated tablet obtained by film-coating the tablet, further more preferably tablet containing compound 1 or a salt thereof, lactose, corn starch, hydroxypropylcellulose, crystalline cellulose, and magnesium stearate or a film-coated tablet obtained by film-coating the tablet, particularly preferably a film-coated tablet obtained by film-coating a tablet containing compound 1 or a salt thereof, lactose, corn starch, hydroxypropylcellulose, crystalline cellulose, and magnesium stearate with hypromellose, macrogol 6 000P, titanium oxide, and magnesium stearate.

In the case of preparing an oral solid preparation, an excipient and optionally a binder, a disintegrant, a lubricant, a colorant, a corrigent, or the like are added to compound 1 or a salt thereof, and a tablet, a film-coated tablet, granules, a powder, a capsule, or the like can then be produced by a routine method.

In the case of preparing an oral liquid preparation, the preparation can be produced by using compound 1 or a salt thereof and a solubilizer, a tonicity agent, a buffer, an antiseptic, or the like.

The daily dose of a drug having the dosage form can be appropriately determined depending on the symptom, body weight, age, sex, or the like of a patient.

Examples

Hereinafter, the present invention will be described in more detail with reference to Examples and Comparative Examples. However, the present invention is not limited by these examples.

Commercially available products were used as various reagents used in Examples and Comparative Examples unless otherwise specified.

The total content of analogues 1 to 9 and their salts contained in a free form of compound 1 was measured by use of high-performance liquid chromatography.

Mass spectra were measured using LCMS-IT-TOF manufactured by Shimadzu Corp. for HRMS and using SQD manufactured by Waters Corp. for LRMS under the following conditions, and [M+H]+ values were shown.

MS detection: ESI positive

Some compounds were structurally confirmed by 1H-NMR, and abbreviations are defined as follows.

s: singlet
d: doublet
t: triplet
q: quartet
sep: septet
dd: doublet of doublets
ddd: doublet of doublet of doublets
m: multiplet
brs: broad singlet

[Reference Example 1] Synthesis of compound 1

Compound 1 was produced by the following production method. (Step 1) Synthesis of 4-iodo-3-(propan-2-yl)-1-(triphenylmethyl)-1H-pyrazolo[3,4-b]pyridine

4-Iodo-3-(propan-2-yl)-1H-pyrazolo[3,4-b]pyridine (6.53 kg) which can be synthesized with reference to Example 100(1) of WO 2011/004610 was dissolved in N,N-dimethylformamide (27.6 kg). To the solution, 66% sodium hydride (0.933 kg) was added at approximately 0°C, and the mixture was stirred for 30 minutes. Trityl chloride (6.66 kg) was added thereto at approximately 0°C, and the mixture was stirred for 30 minutes and then stirred at approximately 25°C for 3.7 hours. A liquid mixture of acetonitrile (14.2 kg), water (12.0 L), and 35% hydrochloric acid (0.24 kg) was added thereto at approximately 40°C, and the mixture was stirred for 30 minutes. Further, a liquid mixture of acetonitrile (4.5 kg) and water (23.0 L) was added thereto at approximately 40°C, and the mixture was cooled to 0°C and stirred for 13.8 hours. Crystals were separated and washed with a liquid mixture of acetonitrile (29.4 kg) and water (15.0 L). The obtained wet crystals were dried under reduced pressure at an outside temperature of 80°C to obtain dry crystals (11.68 kg). The obtained dry crystals (11.6 kg) were added to a liquid mixture of acetonitrile (23.7 kg) and water (12.1 L), and the mixture was heated and stirred at approximately 75°C for 1 hour. The mixture was cooled to 0°C and stirred for 3.5 hours. Crystals were separated and washed with a liquid mixture of acetonitrile (13 L) and water (7.0 kg). The obtained wet crystals were dried under reduced pressure at an outside temperature of 80°C to obtain 4-iodo-3-(propan-2-yl)-1-(triphenylmethyl)-1H-pyrazolo [3,4-b]pyridine.

(Step 2) Synthesis of 4-[4-(1-methyl-1H-pyrazol-4-yl)-1H-imidazol-1-yl]-3-(propan-2-yl)-1-(triphenylmethyl)-1H-pyrazolo[3,4-b]pyridine

Dimethyl sulfoxide (74.0 kg) and 4-(1H-imidazol-4-yl)-1-methyl-1H-pyrazole dihydrochloride (5.99 kg) which can be synthesized with reference to the method described in Non Patent Literature 1 (J. Med. Chem. 2019, 62, 531-551) were dissolved by stirring. Further, sodium t-butoxide (7.73 kg) was added thereto at 25°C, and the mixture was stirred for 10 minutes. Tripotassium phosphate (4.49 kg) was added thereto at approximately 20°C. 4-Iodo-3-(propan-2-yl)-1-(triphenylmethyl)-1H-pyrazolo[3,4-b]pyridine (11.2 kg) was added thereto, and the mixture was stirred at approximately 125°C for 5.5 hours. The reaction solution was cooled to approximately 10°C. A liquid mixture of acetonitrile (24.1 kg) and water (3.77 kg) was added thereto, and the mixture was stirred at approximately 0°C for 2.3 hours. A liquid mixture of water (19.0 L) and 35% hydrochloride acid (4.40 kg) was added thereto at approximately 0°C, and water (120 L) was further added thereto. The mixture was stirred at approximately 5°C for 10.7 hours. Crystals were separated and washed with water (56 L). The whole amount of the obtained wet crystals and water (220 L) were stirred at approximately 35°C for 1 hour. Crystals were separated and washed with water (170 L). The obtained wet crystals were dried under reduced pressure at an outside temperature of 80°C to obtain 4-[4-(1-methyl-1H-pyrazol-4-yl)-1H-imidazol-1-yl]-3-(propan-2-yl)-1-(triphenylmethyl)-1H-pyrazolo[3,4-b]pyridine.

(Step 3) Synthesis of 4-[4-(1-methyl-1H-pyrazol-4-yl)-1H-imidazol-1-yl]-3-(propan-2-yl)-1H-pyrazolo[3,4-b]pyridine

To acetonitrile (23.0 kg), benzyl triethylammonium chloride (2.80 kg), 4-[4-(1-methyl-1H-pyrazol-4-yl)-1H-imidazol-1-yl]-3-(propan-2-yl)-1-(triphenylmethyl)-1H-pyrazolo[3,4-b]pyridine (11.2 kg), and water (0.440 kg) were added, and the mixture was stirred. Methanesulfonic acid (7.83 kg) was added thereto at approximately 40°C, and the mixture was stirred at approximately 45°C for 1.1 hours. Further, water (0.661 kg) was added thereto, and the mixture was stirred at approximately 45°C for 2.7 hours. The reaction solution was cooled. Water (87.0 L) was added thereto at approximately 0°C, and the mixture was stirred for 2 hours. Celite (9.00 kg) was added thereto, and the mixture was stirred. A solid was filtered off, and the residue was washed with water (100 L) and mixed with the filtrate. The obtained mixed solution was cooled, stirred at approximately 0°C for 11.9 hours, and then filtered. The filtrate was added at approximately 20°C to an aqueous sodium carbonate solution prepared from water (49.3 L) and sodium carbonate (7.77 kg), and the mixture was stirred for 3.1 hours. Crystals were separated and washed with water (45.0 L). The obtained wet crystals were dried under reduced pressure at an outside temperature of 80°C to obtain dry crystals (5.42 kg). The obtained dry crystals (4.41 kg) and acetonitrile (35.3 kg) were stirred at approximately 80°C for 2.1 hours. The mixture was cooled and stirred at approximately 5°C for 1 hour. Crystals were separated and washed with acetonitrile (11.0 kg). The obtained wet crystals were dried under reduced pressure at an outside temperature of 80°C to obtain 4-[4-(1-methyl-1H-pyrazol-4-yl)-1H-imidazol-1-yl]-3-(propan-2-yl)-1H-pyrazolo[3,4-b]pyridine (4.26 kg).

(Step 4) Synthesis of compound 1

Dimethyl sulfoxide (29.0 kg), 4-[4-(1-methyl-1H-pyrazol-4-yl)-1H-imidazol-1-yl]-3-(propan-2-yl)-1H-pyrazolo[3,4-b]pyridine (3.91 kg), potassium carbonate (2.11 kg), and 3-ethyl-4-fluorobenzonitrile (5.69 kg) were stirred. The mixture was heated and stirred at approximately 125°C for 5.5 hours. The reaction solution was cooled. Ethyl acetate (79.5 kg) and water (39.0 L) were added thereto, and the mixture was stirred. The mixture was left standing to separate an organic layer and an aqueous layer. To the organic layer, an aqueous sodium chloride solution prepared from sodium chloride (8.80 kg) and water (26.9 L) was added, and the mixture was stirred. The mixture was left standing to separate an organic layer and an aqueous layer. The organic layer was applied to an active carbon filter, and the active carbon filter was washed with ethyl acetate (8.80 kg) and mixed with the filtrate. The mixed solution obtained by mixing the filtrate and the washes was concentrated under reduced pressure to obtain 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzonitrile as a concentrate. To the whole amount of the obtained concentrate, dimethyl sulfoxide (29.0 kg) was added, and the mixture was dissolved by stirring. The solution was cooled. An aqueous sodium hydroxide solution prepared from sodium hydroxide (0.051 kg) and water (0.330 kg) was added thereto at approximately 15°C, and the mixture was stirred. 35% hydrogen peroxide water (3.71 kg) was added thereto at approximately 15°C, and the mixture was stirred. Further, an aqueous sodium hydroxide solution prepared from sodium hydroxide (0.025 kg) and water (0.170 kg) was added thereto at approximately 20°C, and the mixture was stirred. An aqueous sodium thiosulfate solution prepared from sodium thiosulfate pentahydrate (1.39 kg) and water (12.1 L) was added thereto, and the mixture was stirred at approximately 10°C for 2 hours. Further, water (56.2 L) was added thereto, and the mixture was cooled and stirred at approximately 10°C for 16.3 hours. Crystals were separated and washed with water (56.0 L) to obtain crude compound 1 (13.56 kg) as wet crystals.

(Step 5) Purification of compound 1 - 1 -

The obtained crude compound 1 (13.56 kg) was added to methyl acetate (111 kg), and the mixture was stirred. The mixture was heated to reflux and stirred for 10 hours. The mixture was cooled and stirred. The mixture was heated to reflux again and stirred for 2.6 hours. The mixture was cooled and stirred at approximately 10°C for 2.9 hours. Crystals were separated and washed with methyl acetate (16.8 kg). The obtained wet crystals were dried under reduced pressure at an outside temperature of 80°C to obtain purified form 1 of the compound 1 (4.38 kg).

(Step 6) Purification of compound 1 - 2 -

To a liquid mixture of ethanol (59.0 kg) and water (8.30 L), the purified form 1 of the compound 1 (4.17 kg) was added, and the mixture was stirred. Crystals were dissolved by heating at approximately 55°C. The solution was filtered and washed with a liquid mixture of ethanol (5.9 kg) and water (0.84 L), and the filtrate and the washes were mixed. The obtained mixed solution was concentrated under reduced pressure, and water (58.0 L) was then added to the concentrate. The mixture was cooled and stirred at approximately 20°C for 16 hours. Crystals were separated and washed with water (13.0 L). The obtained wet crystals were dried under reduced pressure at an outside temperature of 50°C to obtain purified form 2 of the compound 1 (3.98 kg).

(Step 7) Purification of compound 1 - 3 -

The purified form 2 of the compound 1 (3.00 kg) was added to a liquid mixture of methyl acetate (22.3 kg) and ethanol (4.75 kg), and the mixture was stirred. The mixture was heated to reflux and stirred for 6 hours. Under heating to reflux, methyl acetate (16.7 kg) was added thereto. The mixture was cooled and stirred at approximately 25°C for 11 hours. Crystals were separated and washed with methyl acetate (8.45 kg). The obtained wet crystals were dried under reduced pressure at an outside temperature of 80°C to obtain compound 1 (2.59 kg).

The obtained data on $^1$H-NMR and MS was as follows.
$^1$H-NMR ($CDCl_3$) $\delta$: 8.59 (1H, d, J = 4.8 Hz), 7.97 (1H, d, J = 2.0 Hz), 7.87 (1H, d, J = 1.3 Hz), 7.84-7.75 (3H, m), 7.58 (1H, d, J = 8.1 Hz), 7.39 (1H, d, J = 1.4 Hz), 7.14 (1H, d, J = 4.9 Hz), 6.28 (1H, brs), 5.98 (1H, brs), 3.98 (3H, s), 3.18 (1H, sep, J = 6.8 Hz), 2.61 (2H, q, J = 7.5 Hz), 1.25 (6H, d, J = 6.8 Hz), 1.14 (3H, t, J = 7.6 Hz)

HRMS (ESI) m/z: 455.2310 [M+H]+

[Production Example 1] Synthesis of analogue 1:

Analogue 1 was produced by the following production method. (Step 1) Synthesis of 3-ethyl-4-[4-iodo-3-(propan-2-yl)-1H-pyrazolo[3,4-b]pyridin-1-yl]benzonitrile

3-Ethyl-4-fluorobenzonitrile (6.23 g) was stirred with potassium carbonate (2.31 g) and dimethyl sulfoxide (26.8 mL), and the mixture was warmed to 110°C. 4-Iodo-3-(propan-2-yl)-1H-pyrazolo[3,4-b]pyridine (4 g) which can be

synthesized with reference to Example 100(1) of WO 2011/004610 and dimethyl sulfoxide (0.8 mL) were added thereto, and the mixture was stirred for 17.5 hours and then cooled to room temperature. Ethyl acetate (100 mL), water (20 mL), and 20% saline (10 mL) were added thereto, and the mixture was stirred, followed by the filtration of insoluble matter. After stirring, an aqueous layer was removed to obtain organic layer 1. To the insoluble matter, ethyl acetate (90 mL) and water (50 mL) were added, and the mixture was stirred. Insoluble matter was filtered again, and an aqueous layer was removed to obtain organic layer 2. The organic layer 1 and the organic layer 2 were mixed and concentrated under reduced pressure, and the obtained residue was purified by normal-phase silica gel column chromatography to obtain 3-ethyl-4-[4-iodo-3-(propan-2-yl)-1H-pyrazolo[3,4-b]pyridin-1-yl]benzonitrile (yield: 2.57 g, yield percentage: 44.3%).

(Step 2) Synthesis of 3-ethyl-4-{4-[4-(1-methyl-1H-pyrazol-4-yl)-1H-imidazol-1-yl]-3,3'-di(propan-2-yl)-1'H-[1,4'-bipyrazolo[3,4-b]pyridin]-1'-yl}benzonitrile

4-[4-(1-Methyl-1H-pyrazol-4-yl)-1H-imidazol-1-yl]-3-(propan-2-yl)-1H-pyrazolo[3,4-b]pyridine (2.22 g) obtained in step 3 of Reference Example 1 was stirred with 3-ethyl-4-[4-iodo-3-(propan-2-yl)-1H-pyrazolo[3,4-b]pyridin-1-yl]benzonitrile (2.00 g) obtained in step 1, potassium carbonate (0.7969 g), and dimethyl sulfoxide (12 mL). The mixture was warmed to 130°C and cooled to room temperature 21 hours later. Ethyl acetate (100 mL) and water (50 mL) were added thereto, and the mixture was stirred, followed by the filtration of insoluble matter and the removal of an aqueous layer. To the obtained organic layer, water (50 mL) was added, and the mixture was stirred, followed by the removal of an aqueous layer. The resulting organic layer was dried over sodium sulfate and then concentrated under reduced pressure, and the obtained residue was purified by normal-phase silica gel chromatography and then further purified by reverse-phase chromatography to obtain 3-ethyl-4-{4-[4-(1-methyl-1H-pyrazol-4-yl)-1H-imidazol-1-yl]-3,3'-di(propan-2-yl)-1'H-[1,4'-bipyrazolo[3,4-b]pyridin]-1'-yl}benzonitrile (yield: 735.2 mg, yield percentage: 25.7%).

(Step 3) Synthesis of analogue 1

To 3-ethyl-4-{4-[4-(1-methyl-1H-pyrazol-4-yl)-1H-imidazol-1-yl]-3,3'-di(propan-2-yl)-1'H-[1,4'-bipyrazolo[3,4-b]pyridin]-1'-yl}benzonitrile (650 mg) obtained in step 2, dimethyl sulfoxide (3.25 mL) and sulfolane (1.63 mL) were added, and the mixture was stirred. After cooling to 0°C, a 4 N aqueous sodium hydroxide solution (27.3 μL) and a 30% aqueous hydrogen peroxide solution (334.3 μL) were added thereto, and a 4 N aqueous sodium hydroxide solution (27.3 μL) was further added thereto. A 10% aqueous sodium thiosulfate solution (812.5 μL) was added thereto, and the mixture was warmed to room temperature. Then, 2 N hydrochloric acid (13.5 μL) was added thereto. A 10% aqueous sodium thiosulfate solution (5.27 mL) and ethyl acetate (10 mL) were added thereto, and the mixture was stirred to separate an organic layer and an aqueous layer. To the aqueous layer, ethyl acetate (5 mL) was added, and the mixture was stirred, followed by the removal of an aqueous layer. The organic layers were mixed. Water (5 mL) was added thereto, and the mixture was stirred, followed by the removal of an aqueous layer. The resulting organic layer was dried over sodium sulfate and then concentrated under reduced pressure, and the obtained residue was purified by normal-phase silica gel chromatography. The obtained oil substance was recrystallized with ethanol/water. Crystals were filtered and dried under reduced pressure at 50°C to obtain analogue 1 (yield: 433.3 mg, yield percentage: 64.7%).

The obtained data on $^1$H-NMR and MS was as follows.

$^1$H-NMR ($CD_2Cl_2$) δ: 8.68 (1H, d, J = 4.5 Hz), 8.62 (1H, d, J = 5.0 Hz), 7.94 (1H, d, J = 2.0 Hz), 7.87 (1H, d, J = 1.5 Hz), 7.78-7.80 (3H, m), 7.63 (1H, d, J = 5.0 Hz), 7.58 (1H, d, J = 8.0 Hz), 7.44 (1H, d, J = 1.5 Hz), 7.26 (1H, d, J = 5.0 Hz), 6.35 (1H, brs), 5.82 (1H, brs), 3.94 (3H, s), 3.67 (1H, sep, J = 7.0 Hz), 3.26 (1H, sep, J = 6.5 Hz), 2.65 (2H, q, J = 7.5 Hz), 1.29 (6H, d, J = 7.0 Hz), 1.13 (3H, t, J = 7.5 Hz), 1.12 (6H, d, J = 6.5 Hz) HRMS (ESI) m/z: 614.3095 [M+H]+

[Production Example 2] Synthesis of analogue 2:

To commercially available 3-ethyl-4-fluorobenzonitrile (1.5016 g), dimethyl sulfoxide (18.63 mL) and sulfolane (9.31 mL) were added, and the mixture was stirred. After cooling to 0°C, a 4 N aqueous sodium hydroxide solution (1.383 mL) and a 30% aqueous hydrogen peroxide solution (1.392 mL) were added thereto, and the mixture was stirred at 0°C for 1 hour. A 10% aqueous sodium thiosulfate solution (10 mL) was added thereto, and the mixture was stirred at room temperature. Then, water (24 mL) was added thereto, and the mixture was stirred at room temperature. The precipitate was collected by filtration and washed with water. The resultant was dried under reduced pressure to obtain analogue 2 (yield: 1.1984 g, yield percentage: 71.3%).

The obtained data on $^1$H-NMR and MS was as follows.

$^1$H-NMR ($CDCl_3$) δ: 7.73 (1H, dd, J = 2.0, 7.2 Hz), 7.62 (1H, ddd, J = 2.4, 4.8, 8.4 Hz), 7.06 (1H, dd, J = , 8.8, 9.2 Hz), 5.95 (2H, brs), 2.71 (2H, q, J = 7.6 Hz), 1.25 (3H, t, J = 7.6 Hz)

LRMS (ESI) m/z: 168.2 [M+H]+

[Example 1] Analogue 1 as reference standard

Approximately 25 mg of the compound 1 obtained in Reference Example 1 described above was precisely weighed, dissolved by the addition of a 0.01 mol/l phosphate buffer solution (pH 3.5)/acetonitrile liquid mixture (1:1), and accurately brought to 100 mL to prepare a sample solution. 1 mL of this solution was accurately weighed and accurately brought to 100 mL by the addition of a 0.01 mol/L phosphate buffer solution (pH 3.5)/acetonitrile liquid mixture (1:1) to prepare a standard solution. 10 μL each of the sample solution and the standard solution was accurately weighed and subjected to a test by liquid chromatography under conditions given below. Peak area AT of each individual other than the compound 1 in the sample solution and peak area AS of the compound 1 in the standard solution were measured by the automatic integration method and calculated according to the following equations.

$$\text{Amount (\%) of each individual analogue} = AT \times RF / AS$$

Total amount (%) of analogues = Total sum of the amounts (%) of the individual analogues

RF: sensitivity correction coefficient
Testing conditions
Detector: ultraviolet absorptiometer (measurement wavelength: 220 nm)
Column: a stainless tube having an inside diameter of 4.6 mm and a length of 15 cm was packed with 5 μm of an octadecylsilylated silica gel for liquid chromatography [InertSustain C18 (GL Sciences Inc.) or equivalent].
Column temperature: constant temperature of 40°C
Mobile phase
Mobile phase **A:** 0.01 mol/L phosphate buffer solution (pH 3.5)/acetonitrile liquid mixture (9:1)
Mobile phase **B:** acetonitrile/0.01 mol/L phosphate buffer solution (pH 3.5) liquid mixture (7:3)
Solution sending of mobile phase: the mixing ratio between the mobile phases A and B was changed as described below to control a concentration gradient.
Time (min) after injection Mobile phase A (vol%) Mobile phase B (vol%)

| | | |
|---|---|---|
| 0 to 15 | 72 | 28 |
| 15 to 30 | 72-0 | 28→100 |
| 30 to 40 | 0 | 100 |

Flow rate: 1.0 mL/min
Area measurement range: 40 minutes after injection of the sample solution

System compatibility

Confirmation of detection: 1 mL of the standard solution is accurately weighed and accurately brought to 20 mL by the addition of a 0.01 mol/L phosphate buffer solution (pH 3.5)/acetonitrile liquid mixture (1:1). From 10 μL of this solution, the peak area of the obtained compound 1 is confirmed to be 3.5 to 6.5% of the peak area of the compound 1 in the standard solution.
System performance: when 10 μL of the standard solution is operated under the conditions described above, the number of theoretical plates and symmetry factor of the peak of the compound 1 are 6 000 plates or more and 1.5 or less, respectively.
System reproducibility: when 10 μL of the standard solution is repetitively tested six times under the conditions described above, the relative standard deviation of the peak area of the compound 1 is 2.0% or less.

The compound 1 obtained in Reference Example 1 described above was measured by HPLC in accordance with the method described above.
The results are shown in Figure 1. As a result, a peak was confirmed which exhibited the same retention time as

that of the reference standard of the analogue 1 (Figure 2). An analogue was obtained by separating the peak which exhibited the same retention time as that of the reference standard of the analogue 1 (Figure 2) contained in the compound 1, and its $^1$H-NMR measurement was carried out with the reference standard of the analogue 1 (Figure 2). As a result, these analogues were confirmed to be the same component.

Accordingly, the analogue 1 was found to be useful as a reference standard for confirming the quality of API of the compound 1. From the results of Figure 1, the content of the analogue 1 in the compound 1 was confirmed to be 0.3% or less.

A film-coated tablet containing the compound 1 obtained by the same production method as in Reference Example 1 and having the composition shown in Table 2 was prepared, and the content of the analogue 1 therein was measured and consequently confirmed to be 0.3% or less.

[Table 2]

| | Film-coated tablet |
|---|---|
| <Plain tablet> | |
| Compound 1 | 40 |
| Lactate hydrate | 38.5 |
| Corn starch | 15 |
| Hydroxypropylcellulose | 3 |
| Crystalline cellulose | 52.75 |
| Magnesium stearate | 0.75 |
| <Film coating layer> | |
| Hypromellose | 3.6 |
| Macrogol 6000P | 0.45 |
| Titanium oxide | 0.45 |
| Magnesium stearate | 0.0045 |
| Total (mg) | 154.5045 |

[Example 2] Analogue 2 as reference standard

The compound 1 obtained in Reference Example 1 described above was measured by HPLC in accordance with the method described in Example 1 (Figure 1). As a result, a peak was confirmed which exhibited the same retention time as that of the reference standard of the analogue 2 (Figure 3).

Accordingly, the analogue 2 was found to be useful as a reference standard for confirming the quality of API of the compound 1. From the results of Figure 1, the content of the analogue 2 in the compound 1 was able to be confirmed to be 0.1% or less. All of the contents of the analogues other than the analogues 1 and 2 were 0.1% or less.

A film-coated tablet containing the compound 1 obtained by the same production method as in Reference Example 1 and having the composition shown in Table 2 was prepared, and the content of the analogue 2 therein was measured and was consequently able to be confirmed to be 0.1% or less. All of the contents of the analogues other than the analogues 1 and 2 were 0.1% or less.

## Claims

**1.** An analogue or a salt thereof that is any of the following analogues 1 to 9 or salts thereof, or a combination of the analogue(s) and the salt(s), for use as a reference standard for controlling the quality of 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a salt thereof:

analogue 1: 3-ethyl-4-{4-[4-(1-methyl-1H-pyrazol-4-yl)-1H-imidazol-1-yl]-3,3'-di(propan-2-yl)-1'H-[1,4'-bipyrazolo[3,4-b]pyridin]-1'-yl}benzamide;
analogue 2: 3-ethyl-4-fluorobenzamide;
analogue 3: N-[1-(4-carbamoyl-2-ethylphenyl)-3-(propan-2-yl)-1H-pyrazolo[3,4-b]pyridin-4-yl]-3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide;
analogue 4: 3-ethyl-4-{$1^4$-[4-(1-methyl-1H-pyrazol-4-yl)-1H-imidazol-1-yl]-$1^3$,$2^3$,$3^3$-tri(propan-2-yl)-$3^1$H-[$1^1$,$2^4$:$2^1$,$3^4$-terpyrazolo[3,4-b]pyridin]-$3^1$-yl}benzamide;
analogue 5: 4,4'-(1H,1'H-[4,4'-biimidazole]-1,1'-diylbis{[3-(propan-2-yl)-1H-pyrazolo[3,4-b]pyridine-4,1-diyl]})bis(3-ethylbenzamide);
analogue 6: 4-{4,6-bis[4-(1-methyl-1H-pyrazol-4-yl)-1H-imidazol-1-yl]-3-(propan-2-yl)-1H-pyrazolo[3,4-b]pyridin-1-yl}-3-ethylbenzonitrile;
analogue 7: 4-{4,6-bis[4-(1-methyl-1H-pyrazol-4-yl)-1H-imidazol-1-yl]-3-(propan-2-yl)-1H-pyrazolo[3,4-b]pyridin-1-yl}-3-ethylbenzamide;
analogue 8: 4-[4-ethoxy-3-(propan-2-yl)-1H-pyrazolo[3,4-b]pyridin-1-yl]-3-ethylbenzamide; and
analogue 9: 3-ethyl-4-[4-methoxy-3-(propan-2-yl)-1H-pyrazolo[3,4-b]pyridin-1-yl]benzamide.

**2.** The analogue or a salt thereof, or a combination of the analogue(s) and the salt(s) according to claim 1, wherein the analogue for use as a reference standard is any of the analogues 1 to 7.

**3.** The analogue or a salt thereof, or a combination of the analogue(s) and the salt(s) according to claim 1, wherein the analogue for use as a reference standard is any of the analogues 1 to 3.

**4.** The analogue or a salt thereof, or a combination of the analogue(s) and the salt(s) according to claim 1, wherein the analogue for use as a reference standard is any of the analogues 1 and 2.

**5.** The analogue or a salt thereof, or a combination of the analogue(s) and the salt(s) according to claim 1, wherein the analogue for use as a reference standard is the analogue 1.

**6.** A pharmaceutical composition for oral administration, comprising 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a salt thereof as an active ingredient, wherein a total content of the following analogues 1 to 9 is 1.0% or less:

analogue 1: 3-ethyl-4-{4-[4-(1-methyl-1H-pyrazol-4-yl)-1H-imidazol-1-yl]-3,3'-di(propan-2-yl)-1'H-[1,4'-bipyrazolo[3,4-b]pyridin]-1'-yl}benzamide;
analogue 2: 3-ethyl-4-fluorobenzamide;
analogue 3: N-[1-(4-carbamoyl-2-ethylphenyl)-3-(propan-2-yl)-1H-pyrazolo[3,4-b]pyridin-4-yl]-3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide;
analogue 4: 3-ethyl-4-{$1^4$-[4-(1-methyl-1H-pyrazol-4-yl)-1H-imidazol-1-yl]-$1^3$,$2^3$,$3^3$-tri(propan-2-yl)-3'H-[$1^1$,$2^4$:$2^1$,$3^4$-terpyrazolo[3,4-b]pyridin]-$3^1$-yl}benzamide;
analogue 5: 4,4'-(1H,1'H-[4,4'-biimidazole]-1,1'-diylbis{[3-(propan-2-yl)-1H-pyrazolo[3,4-b]pyridine-4,1-diyl]})bis(3-ethylbenzamide);
analogue 6: 4-{4,6-bis[4-(1-methyl-1H-pyrazol-4-yl)-1H-imidazol-1-yl]-3-(propan-2-yl)-1H-pyrazolo[3,4-b]pyridin-1-yl}-3-ethylbenzonitrile;
analogue 7: 4-{4,6-bis[4-(1-methyl-1H-pyrazol-4-yl)-1H-imidazol-1-yl]-3-(propan-2-yl)-1H-pyrazolo[3,4-b]pyridin-1-yl}-3-ethylbenzamide;
analogue 8: 4-[4-ethoxy-3-(propan-2-yl)-1H-pyrazolo[3,4-b]pyridin-1-yl]-3-ethylbenzamide; and
analogue 9: 3-ethyl-4-[4-methoxy-3-(propan-2-yl)-1H-pyrazolo[3,4-b]pyridin-1-yl]benzamide.

**7.** The pharmaceutical composition for oral administration according to claim 6, wherein a content of the analogue 1 is 0.30% or less, and all of individual contents of the analogues 2 to 9 are 0.10% or less.

**8.** A pharmaceutical composition for oral administration, comprising 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide as an active ingredient, wherein a content of 3-ethyl-4-{4-[4-(1-methyl-1H-pyrazol-4-yl)-1H-imidazol-1-yl]-3,3'-di(propan-2-yl)-1'H-[1,4'-bipyrazolo[3,4-b]pyridin]-1'-yl}benzamide as analogue 1 is 0.30% or less.

**9.** A pharmaceutical preparation for oral administration, comprising the pharmaceutical composition for oral administration according to any one of claims 6 to 8.

**10.** 3-Ethyl-4-{4-[4-(1-methyl-1H-pyrazol-4-yl)-1H-imidazol-1-yl]-3,3'-di(propan-2-yl)-1'H-[1,4'-bipyrazolo[3,4-b]pyridin]-1'-yl}benzamide or a salt thereof.

**11.** 3-Ethyl-4-fluorobenzamide or a salt thereof.

**12.** N-[1-(4-Carbamoyl-2-ethylphenyl)-3-(propan-2-yl)-1H-pyrazolo[3,4-b]pyridin-4-yl]-3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a salt thereof.

**13.** 3-Ethyl-4-{$1^4$-[4-(1-methyl-1H-pyrazol-4-yl)-1H-imidazol-1-yl]-$1^3$,$2^3$,$3^3$-tri(propan-2-yl)-$3^1$H-[$1^1$,$2^4$:$2^1$,$3^4$-terpyrazolo[3, 4-b]pyridin] - $3^1$-yl}benzamide or a salt thereof.

**14.** 4,4'-(1H,1'H-[4,4'-Biimidazole]-1,1'-diylbis{[3-(propan-2-yl)-1H-pyrazolo[3,4-b]pyridine-4,1-diyl]})bis(3-ethylbenzamide) or a salt thereof.

**15.** 4-{4,6-Bis[4-(1-methyl-1H-pyrazol-4-yl)-1H-imidazol-1-yl]-3-(propan-2-yl)-1H-pyrazolo[3,4-b]pyridin-1-yl}-3-ethylbenzonitrile or a salt thereof.

**16.** 4-{4,6-Bis[4-(1-methyl-1H-pyrazol-4-yl)-1H-imidazol-1-yl]-3-(propan-2-yl)-1H-pyrazolo[3,4-b]pyridin-1-yl}-3-ethylbenzamide or a salt thereof.

**17.** 4-[4-Ethoxy-3-(propan-2-yl)-1H-pyrazolo[3,4-b]pyridin-1-yl]-3-ethylbenzamide or a salt thereof.

**18.** 3-Ethyl-4-[4-methoxy-3-(propan-2-yl)-1H-pyrazolo[3,4-b]pyridin-1-yl]benzamide or a salt thereof.

**19.** Use of any of the following analogues 1 to 9 or salts thereof, or a combination of the analogue(s) and the salt(s) as a reference standard for controlling the quality of 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a salt thereof:

analogue 1: 3-ethyl-4-{4-[4-(1-methyl-1H-pyrazol-4-yl)-1H-imidazol-1-yl]-3,3'-di(propan-2-yl)-1'H-[1,4'-bipyrazolo[3,4-b]pyridin]-1'-yl}benzamide;
analogue 2: 3-ethyl-4-fluorobenzamide;
analogue 3: N-[1-(4-carbamoyl-2-ethylphenyl)-3-(propan-2-yl)-1H-pyrazolo[3,4-b]pyridin-4-yl]-3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide;
analogue 4: 3-ethyl-4-{$1^4$-[4-(1-methyl-1H-pyrazol-4-yl)-1H-imidazol-1-yl]-$1^3$,$2^3$,$3^3$-tri(propan-2-yl)-$3^1$H-[$1^1$,$2^4$:$2^1$,$3^4$-terpyrazolo[3,4-b]pyridin]-$3^1$-yl}benzamide;
analogue 5: 4,4'-(1H,1'H-[4,4'-biimidazole]-1,1'-diylbis{[3-(propan-2-yl)-1H-pyrazolo[3,4-b]pyridine-4,1-diyl]})bis(3-ethylbenzamide);
analogue 6: 4-{4,6-bis[4-(1-methyl-1H-pyrazol-4-yl)-1H-imidazol-1-yl]-3-(propan-2-yl)-1H-pyrazolo[3,4-b]pyridin-1-yl}-3-ethylbenzonitrile;
analogue 7: 4-{4,6-bis[4-(1-methyl-1H-pyrazol-4-yl)-1H-imidazol-1-yl]-3-(propan-2-yl)-1H-pyrazolo[3,4-b]pyridin-1-yl}-3-ethylbenzamide;
analogue 8: 4-[4-ethoxy-3-(propan-2-yl)-1H-pyrazolo[3,4-b]pyridin-1-yl]-3-ethylbenzamide; and
analogue 9: 3-ethyl-4-[4-methoxy-3-(propan-2-yl)-1H-pyrazolo[3,4-b]pyridin-1-yl]benzamide.

**20.** The use according to claim 19, wherein the analogue is any of the analogues 1 to 7.

**21.** The use according to claim 19, wherein the analogue is any of the analogues 1 to 3.

**22.** The use according to claim 19, wherein the analogue is any of the analogues 1 and 2.

**23.** The use according to claim 19, wherein the analogue is the analogue 1.

Fig. 1

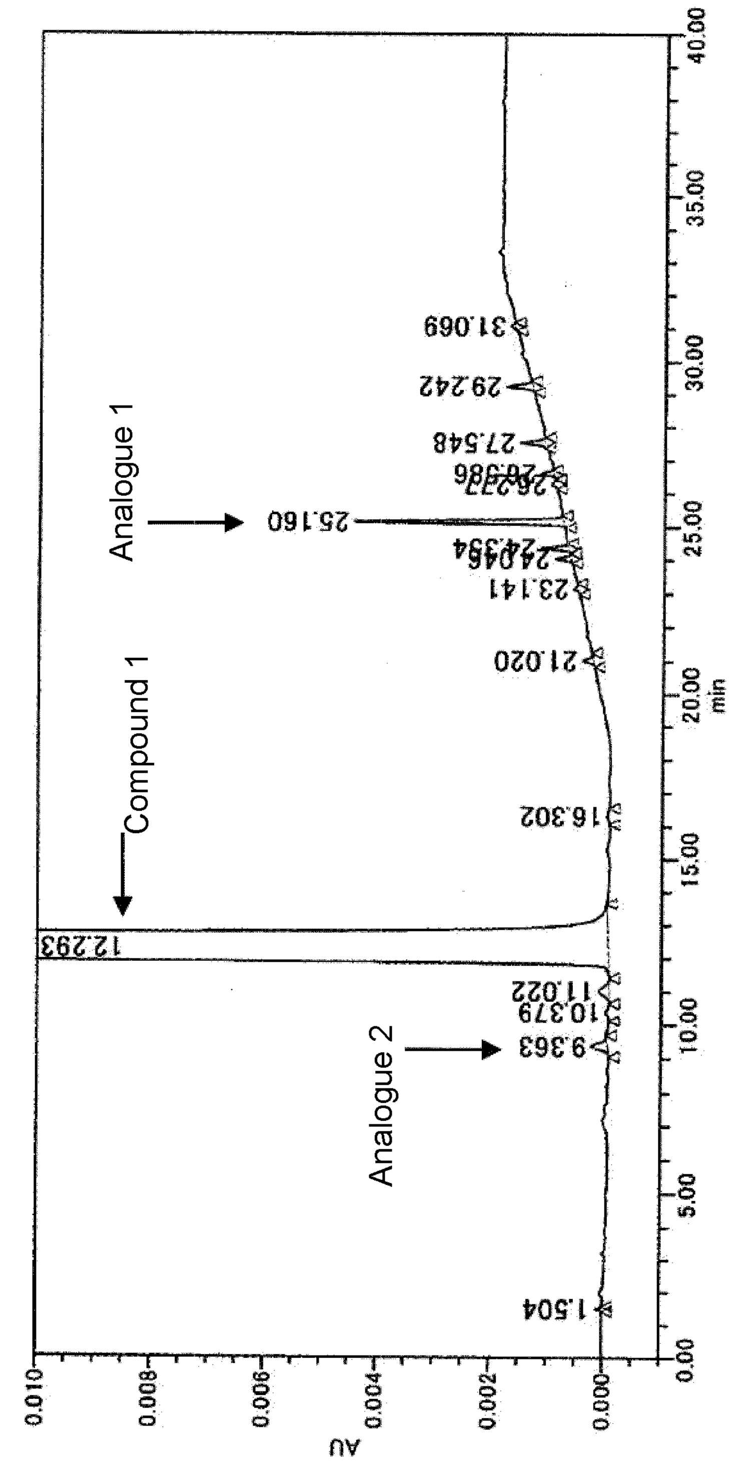
Analogue 1
Compound 1
Analogue 2
1.504
9.363
10.379
11.022
12.293
16.302
21.020
23.141
24.046
24.354
25.160
26.273
26.986
27.548
29.242
31.069
AU
0.010
0.008
0.006
0.004
0.002
0.000
min
0.00
5.00
10.00
15.00
20.00
25.00
30.00
35.00
40.00

Fig. 2

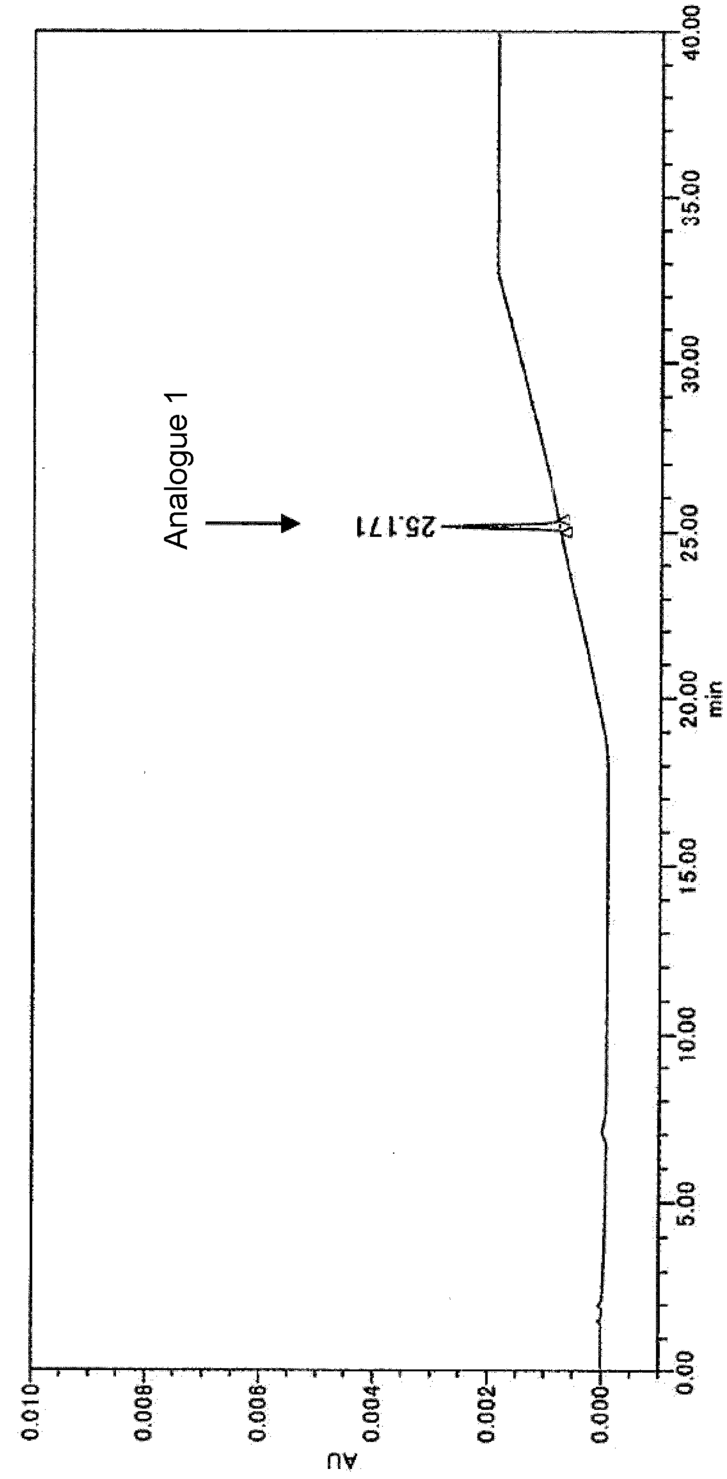
Analogue 1
25.171
AU
0.010
0.008
0.006
0.004
0.002
0.000
min
0.00
5.00
10.00
15.00
20.00
25.00
30.00
35.00
40.00

Fig. 3

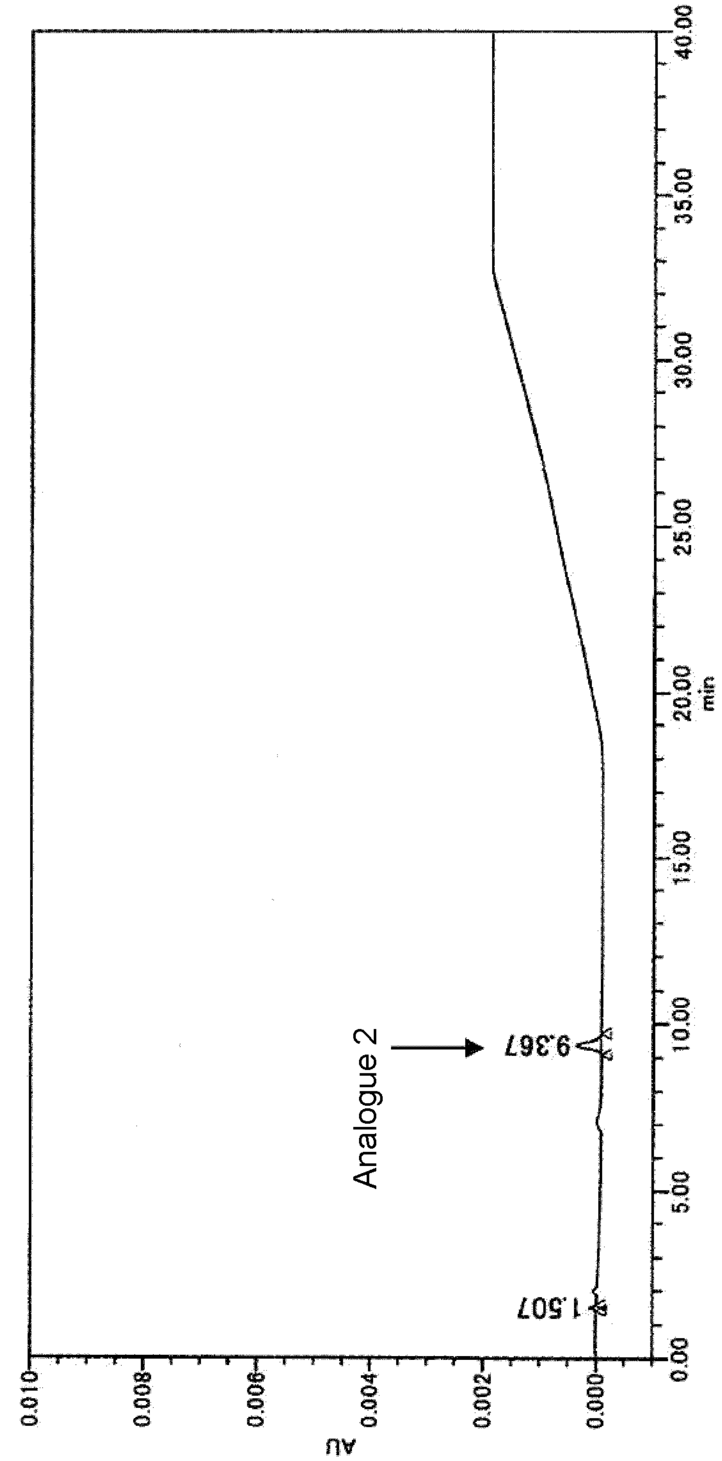
Analogue 2
9.367
1.507
AU
0.010
0.008
0.006
0.004
0.002
0.000
min
0.00
5.00
10.00
15.00
20.00
25.00
30.00
35.00
40.00

## INTERNATIONAL SEARCH REPORT

International application No.
**PCT/JP2023/021536**

**A. CLASSIFICATION OF SUBJECT MATTER**

***C07C 233/65***(2006.01)i; ***A61K 31/166***(2006.01)i; ***A61K 31/437***(2006.01)i; ***A61P 35/00***(2006.01)i; ***C07D 471/04***(2006.01)i; ***C07D 519/00***(2006.01)i
FI: C07C233/65 CSP; A61K31/166; A61K31/437; A61P35/00; C07D471/04 106C; C07D519/00 311

According to International Patent Classification (IPC) or to both national classification and IPC

**B. FIELDS SEARCHED**

Minimum documentation searched (classification system followed by classification symbols)

C07C233/65; A61K31/166; A61K31/437; A61P35/00; C07D471/04; C07D519/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X<br>A | JP 2010-535192 A (PFIZER INC.) 18 November 2010 (2010-11-18)<br>paragraphs [0482], [0483] | 1-4, 11<br>5-10, 12-23 |
| X<br>Y | WO 2016/181990 A1 (TAIHO PHARMACEUTICAL CO., LTD.) 17 November 2016 (2016-11-17)<br>claims, examples, test examples, table 3 | 6–9<br>1-23 |
| Y | YOSHIMURA, Chihoko et al. Thermodynamic Dissection of Potency and Selectivity of Cytosolic Hsp90 Inhibitors, J. Med. Chem., 23 February 2021, vol. 64, no. 5, pp. 2669-2677, Internet: <URL: https://doi.org/10.1021/acs.jmedchem.0c01715><br>abstract, introduction, fig. 1 | 1-23 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 July 2023** | **01 August 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br>Telephone No. |

Form PCT/ISA/210 (second sheet) (January 2015)

**INTERNATIONAL SEARCH REPORT**

International application No. **PCT/JP2023/021536**

**C. DOCUMENTS CONSIDERED TO BE RELEVANT**

| Category* | Citation of document, with indication, where appropriate, of the relevant passages | Relevant to claim No. |
|---|---|---|
| Y | 厚生労働省医薬局審査管理課長. 新有効成分含有医薬品のうち原薬の不純物に関するガイドラインの改定について. 医薬審発第1216001号, 16 December 2002, Internet: <URL: https://www.pmda.go.jp/files/000156108.pdf>, non-official translation (DIRECTOR OF EVALUATION AND LICENSING DIVISION, PHARMACEUTICAL AND FOOD SAFETY BUREAU, MINISTRY OF HEALTH, LABOUR, AND WELFARE. Guideline Concerning Active Ingredient Impurities in New Active Component-Containing Drugs. PFSB / ELD Notification No. 1216001.) in particular, 3.1. Organic Impurities, 5. Report on Amount of Impurities in Lots, 6. Impurities to Be Set in Standards | 1-23 |
| Y | 山下和子等. オンライン濃縮前処理装置付きミクロ液体クロマトグラフ質量分析システム－農医薬関連化合物の微量不純物構造解析への挑戦－. 住友化学. 2002, pp. 56-64, (YAMASHITA, Kazuko et al. Development of an On-line Sample Enrichment System Coupled to ESI-TOFMS - Challenge to highly sensitive structural elucidation of impurities of agrochemicals and pharmaceuticals -. Sumitomo Kagaku [Sumitomo Chemical].) p. 56, left column, lines 1-8 | 1-23 |
| Y | 村上智教等. HPLCハイフネーテッド技術による医薬品の不純物の構造決定. CHROMATOGRAPHY. 2012, vol. 33, no. 3, pp. 179-190, (MURAKAMI, Tomonori, FUKUTSU, Naoto. Structure Elucidation of Impurities in Pharmaceuticals by HPLC Hyphenated Techniques.) p. 179, left column, line 1 to p. 180, left column, line 2 | 1-23 |
| Y | 宇野貴夫等. 第37回メディシナルケミストリーシンポジウム 優秀賞 受賞. 新規経口型HSP90阻害剤TAS-116の創製. MEDCHEM NEWS. 2020, vol. 30, no. 3, pp. 133-136, Internet: <URL: https://doi.org/10.14894/medchem.30.3_133>, (UNO, Takao et al. Discovery of TAS-116 as a novel orally available HSP90 inhibitor), non-official translation (Selected as Top Paper, 37th Medicinal Chemistry Symposium.) p. 136, in particular, 6. Conclusion | 1-23 |

Form PCT/ISA/210 (second sheet) (January 2015)

**INTERNATIONAL SEARCH REPORT**
**Information on patent family members**

International application No.
**PCT/JP2023/021536**

| Patent document cited in search report | Publication date (day/month/year) | Patent family member(s) | Publication date (day/month/year) |
|---|---|---|---|
| JP 2010-535192 A | 18 November 2010 | EP 2185537 A1<br>p. 138<br>KR 10-2010-0045497 A<br>CN 101815710 A | |
| WO 2016/181990 A1 | 17 November 2016 | US 2018/0148443 A1<br>claims, examples, table 3<br>EP 3296299 A1<br>CN 107531707 A<br>KR 10-2018-0003558 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011004610 A
- WO 2015046498 A
- WO 2019004417 A
- WO 2016181990 A
- WO 2019054465 A


### Non-patent literature cited in the description


- *J. Med. Chem.*, 2019, vol. 62, 531-551